# EUROPEAN PATENT APPLICATION

(11) **EP 4 763 283 A2**
(43) Date of publication of application: **24.06.2026**
(21) Application number: 26174936.0
(22) Date of filing: 24.02.2021
(51) Int. Cl.: A61P 23/00

(54) **EUTECTIC BASED ANESTHETIC COMPOSITIONS AND APPLICATIONS THEREOF**

(30) Priority: 25.02.2020 US 202062981273 P
(62) Divisional of application: 21761615.0
(71) Applicant: The University of North Carolina at Chapel Hill, Chapel Hill, NC 27514 (US)
(72) Inventor: JAY, Michael, Chapel Hill, NC 27599-7355 (US); ZELDES, Ben, Chapel Hill, NC 27599-7355 (US); MARTIN, Jesse, Chapel Hill, NC 27599-7355 (US); MCMILLAN, Stephen, Chapel Hill, NC 27599-7355 (US)
(74) Representative: Greenwoods

(57) **Abstract**

In view of the health and addiction risks associated with opiates, anesthetic emulsions are described herein comprising eutectic mixtures of local anesthetics. In some embodiments, the anesthetic emulsions release one or more local anesthetics over a period of several days for continuous pain relief. An anesthetic emulsion, in some embodiments, comprises a dispersed phase comprising a eutectic mixture of menthol lidocaine and benzocaine, and an aqueous or aqueous-based continuous phase comprising bupivacaine.

## Description

### CROSS REFERENCE TO RELATED APPLICATIONS

The present application claims priority to U.S. Provisional Application No. 62/981,273 filed February 25, 2020.

### STATEMENT REGARDING FEDERALLY SPONSORED RESEARCH

This invention was made with government support under Grant No. DE028208 awarded by the National Institutes of Health. The government has certain rights in the invention.

### FIELD

The present disclosure relates to anesthetic compositions and, in particular, to non-opiate anesthetic emulsions comprising eutectic mixtures.

### BACKGROUND

A need exists for an intermediate-length pain management option for dealing with dental and/or other surgical pain. Topical and injected local anesthetics are highly effective at managing pain during and shortly after procedures, and in most cases subsequent pain is mild. However, tooth extractions can result in prolonged pain, particularly in cases of "dry-socket" - intense, sometimes radiating pain caused by loss of the blood clot from the alveolar (tooth) socket. Dry-socket pain presents one to three days after extraction, can last up to 10 days, and occurs in a significant number of third-molar (wisdom tooth) extractions, although any tooth extraction carries some risk. Currently, opioids are frequently prescribed following third-molar tooth extractions as a post-operative care strategy to manage acute pain. Furthermore, since third molars are typically removed in adolescence or young adulthood, this is the first exposure to opioids for many patients, and results in an increased risk for persistent opioid use and abuse.

The Centers for Disease Control and Prevention (CDC) reported that in 2018, 67,367 drug overdose deaths occurred in the United States, mostly (70%) driven by synthetic opioids. Dental practitioners are among the leading prescribers of opioid prescriptions, with 85% of oral surgeons indicating they nearly always prescribe opiates after third molar extraction to reduce acute pain. Approximately 21 million surgical tooth extractions are performed on 10 million patients each year in the U.S. (Survey of Dental Services Rendered, American Dental Association).

### SUMMARY

In view of the serious health and addiction risks associated with opiates, anesthetic emulsions are described herein comprising eutectic mixtures. In some embodiments, the anesthetic emulsions release one or more local anesthetics over a period of several days for continuous pain relief. An anesthetic emulsion, in some embodiments, comprises a dispersed phase comprising a eutectic mixture of lidocaine and benzocaine, and an aqueous or aqueous-based continuous phase comprising bupivacaine. A ratio of lidocaine to benzocaine, in some embodiments, ranges from 1.5 to 3. Moreover, in some embodiments, lidocaine can be present in an amount of 10-20 weight percent of the anesthetic emulsion, while benzocaine can be present in an amount of 3-8 weight percent of the anesthetic emulsion. Moreover, in some embodiments, bupivacaine is present in an amount up to 5 weight percent of the anesthetic emulsion. Bupivacaine, for example, can be present in an amount of 0.5-3.0 weight percent, in some embodiments.

In some embodiments, the eutectic mixture further comprises menthol. Menthol can be present in any desired amount consistent with the technical objectives described herein. Menthol, for example, can be present in an amount of 0.5 to 2 weight percent of the anesthetic emulsion, in some embodiments. The aqueous or aqueous-based continuous phase may further comprise one or more gelling agents. In some embodiments, one or more gelling agents are present in a total amount of 15-25 weight percent of the anesthetic emulsion. Gelling agents can comprise block copolymers comprising hydrophobic and hydrophilic blocks, in some embodiments. Gelling agents can be employed to adjust viscosity of the anesthetic emulsion. The anesthetic emulsion, for example, can have a viscosity of 3-5 Pa-s at 30°C at a shear rate of 500/s.

In another aspect, methods of treating sites of damaged tissue are described herein. A method comprises applying an anesthetic emulsion to the site of damaged tissue, the anesthetic emulsion comprising a dispersed phase comprising a eutectic mixture of lidocaine and benzocaine, and an aqueous or aqueous-based continuous phase comprising bupivacaine. In some embodiments, a method further comprises releasing at least one of the lidocaine, benzocaine, and bupivacaine to the site of damaged tissue over a period of at least three days. The site of damaged tissue can be a surgical site. In some embodiments, the surgical site resides in a patient's mouth, such as a tooth socket that is formed after an extraction procedure (e.g., molar extraction). The method can be performed to treat the tooth socket after dental extraction and/or as the surgical site is healing. For instance, the method can be used as a preventative to reduce the chances of a dry socket forming and/or as a treatment at the surgical site if a dry socket forms. Alternatively, the site of damaged tissue is a site of injury. A site of injury can include one or more cuts/lacerations, for example. Anesthetic emulsions employed in methods of treating sites of damaged tissue can have any composition and/or properties described herein.

These and other embodiments are further described in the following detailed description with reference to the Drawings filed herewith.

### BRIEF DESCRIPTION OF THE DRAWINGS

FIG. 1 illustrates a series of graphs depicting heat flow measurements for different example lidocaine-benzocaine mixtures over a temperature range.
FIG. 2A illustrates a graph depicting temperature vs. weight fraction lidocaine for an example lidocaine-benzocaine system.
FIG. 2B illustrates a graph depicting temperature vs. weight fraction benzocaine for an example benzocaine-menthol system.
FIG. 3 illustrates a ternary phase diagram for a lidocaine-benzocaine-menthol system.
FIG. 4A illustrates a graph depicting viscosity vs. shear rate for example systems.
FIG. 4B illustrates a graph depicting viscosity vs. temperature for example systems.
FIG. 5A illustrates a series of graphs depicting soluble drug stability of benzocaine (top), lidocaine (middle), and bupivacaine HCl (bottom) under different conditions.
FIG. 5B illustrates a series of graphs depicting formulation drug stability of benzocaine (top), lidocaine (middle), and bupivacaine HCl (bottom) under different conditions.
FIG. 6 illustrates a graph depicting drug release over time for an example composition in accordance with the present disclosure.
FIG. 7 depicts an example composition in accordance with example formulations of the present disclosure.

### DETAILED DESCRIPTION

Embodiments described herein can be understood more readily by reference to the following detailed description and examples and their previous and following descriptions. Elements, apparatus and methods described herein, however, are not limited to the specific embodiments presented in the detailed description and examples. It should be recognized that these embodiments are merely illustrative of the principles of the present disclosure. Numerous modifications and adaptations will be readily apparent to those of skill in the art without departing from the spirit and scope of the disclosure.

In one aspect, in addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "from 5 to 10" or "5-10" should generally be considered to include the end points 5 and 10.

Further, when the phrase "up to" is used in connection with an amount or quantity, it is to be understood that the amount is at least a detectable amount or quantity. For example, a material present in an amount "up to" a specified amount can be present from a detectable amount and up to and including the specified amount.

As described herein, an anesthetic emulsion, in some embodiments, comprises a dispersed phase comprising a eutectic mixture of lidocaine and benzocaine, and an aqueous or aqueous-based continuous phase comprising bupivacaine. A ratio of lidocaine to benzocaine, in some embodiments, ranges from 1.5 to 3. Moreover, in some embodiments, lidocaine can be present in an amount of 10-20 weight percent of the anesthetic emulsion, while benzocaine can be present in an amount of 3-8 weight percent of the anesthetic emulsion. Moreover, in some embodiments, bupivacaine is present in an amount up to 5 weight percent of the anesthetic emulsion. Bupivacaine, for example, can be present in an amount of 0.5-3.0 weight percent, in some embodiments.

Anesthetic emulsions contemplated by the present application are further illustrated by the following non-limiting examples.

### EXAMPLE 1 - Anesthetic Emulsion

In one formulation, which is an oil-in-water emulsion, the oil phase includes a eutectic mixture of three solid materials: two primary anesthetics (lidocaine and benzocaine) and menthol.

A eutectic mixture is defined as a mixture of two or more components which usually do not interact to form a new chemical compound but, which at certain ratios, inhibit the crystallization process of one another resulting in a system having a lower melting point than either of the components.

The present oily eutectic mixture comprises two primary anesthetics, lidocaine (67.5% wt of the oil phase) and benzocaine (27.5% wt of the oil phase) as well as a small amount of menthol (5% wt of the oil phase). This three-component eutectic, designated as LiBeMe, makes up 20% wt of the final formulation and is emulsified in an aqueous phase containing gelling agents, surfactants, a preservative, and a soluble anesthetic (bupivacaine hydrochloride). Another component of the formulation, Pluronic F127 (Poloxamer-407), is a gelling agent that possesses unique thermogelling and surfactant properties. The low toxicity and resorbable nature of Pluronics in general mean they may be used clinically as a replacement for 'bone wax' (a non-resorbable hemostatic agent), where they have been found not to interfere with osteogenesis. Osteogenesis is important for a potential tooth socket filling, since the socket needs to gradually fill with bone as it heals.

The composition of the formulation is provided in Table 1.

**Table 1: Composition of the Anesthetic Emulsion Formulation.**

| **Component** | **Wt/wt in LBB-111** | **Role** |
|---|---|---|
| Pluronic F127 (Poloxamer 407) | 16% | Gelling agent, weak surfactant |
| Lidocaine | 13.5% | Local anesthetic |
| Benzocaine | 5.5% | Local anesthetic |
| Carboxymethylcellulose-sodium (CMC) | 2% | Gelling agent |
| Bupivacaine-HCl | 1% | Local anesthetic (long acting) |
| Menthol | 1% | Eutectic stabilizer, flavor |
| Polysorbate 80 (Tween 80) | 0.05% | Surfactant |
| Disodium-EDTA | 0.05% | Preservative |
| Water | 60.45% | |

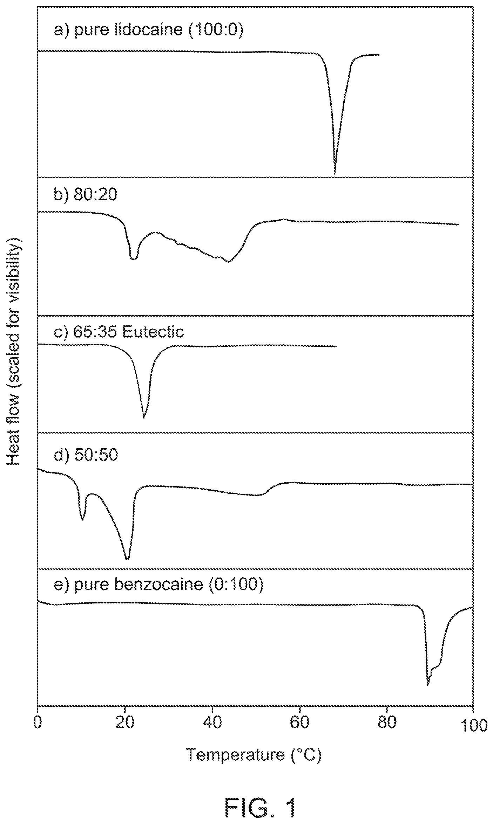

LBB-111 is an oil-in-water emulsion in which the oil phase comprises a eutectic mixture of two solid anesthetics, lidocaine (67.5% of the weight of the oil phase) and benzocaine (27.5% of the weight of the oil phase as well as a small amount of menthol (5% of the weight of the oil phase) **(****Fig 1****).** This three-component eutectic, designated as LiBeMe, makes up 20% of the weight of the final formulation and is emulsified in an aqueous phase containing gelling agents, surfactants, a preservative, and a third, soluble anesthetic (bupivacaine hydrochloride). Another component of the formulation, Pluronic F127 (Poloxamer-407), confers the sustained release properties of the anesthetic agents over time.

Menthol helps maintain the stability of the eutectic as well as to enhance the flavor. It does not lower the melting point, but rather prevents nucleation and allows the oil phase to remain as a supercooled liquid well below the measured freezing temperature. Polysorbate 80 and carboxymethyl cellulose (CMC) help prevent separation and creaming, especially if the emulsion is stored for long periods of time at lower temperatures. The properties of the three anesthetics are listed in Table 2.

**Table 2: Physiochemical Properties and Activity Profile of Selected Local Anesthetics.**

| | 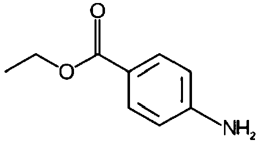 | 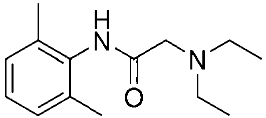 | 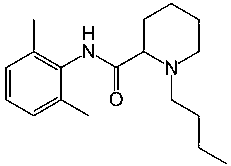 |
|---|---|---|---|
| | Benzocaine | Lidocaine | Bupivacaine |
| pKa | 2.8 | 7.9 | 8.1 |
| logP | 1.86 | 2.30 | 3.41 |
| logD_{7.4} | 1.86 | 1.75 | 2.39 |
| Onset | Fast | Fast | Moderate |
| Potency | Low | Medium | High |
| Duration | Short | Moderate | Long |

### Preparation of Formulation

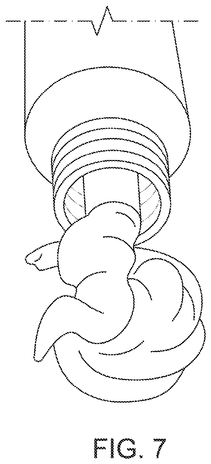

The anesthetic emulsion included (by weight): 20% LiBeMe eutectic, 16% Pluronic F127, 2% CMC, 1% Bupivacaine-HCl, 0.5% Tween 80, 0.05% Na₂-EDTA, with the rest made up of water. Stock solutions consisted of liquid LiBeMe (67.5% lidocaine, 27.5% benzocaine, 5% menthol) melted in a 40°C incubator, 35% F127 added to water that already had 2.2% Bupivacaine HCl at 1.1% Tween - liquefied in a freezer, 10% CMC in water partially mixed by vortexing then homogenized by syringe mixing, and 5% Na₂-EDTA pH adjusted to 7.5 in water using 10 M NaOH. Appropriate amounts (Pluronic F127 solution to 60% of the final mass, 20% each of CMC and LiBeMe) of all stock solutions were weighed into an Unguator jar. The mixing shaft was inserted through the jar's lid and a disposable mixing blade attached. The lid was loosely screwed on to the jar and excess air removed. The jar was affixed to the Unguator and mixed using the recommended emulsion settings (30 sec at 600 rpm, followed by 30 sec at 1900 rpm) for three cycles, with consistent up and down mixing throughout. The final anesthetic emulsion was a white cream, as exemplified in FIG. 7.

*Materials:* From Spectrum Chemical: Pluronic F127 (P1166), lidocaine (LI102), Benzocaine (BE130), Menthol (ME110), Bupivacaine-HCl monohydrate (B1391), Polysorbate 80 (PO138). From Sigma Aldrich: carboxymethylcellulose (CMC) sodium salt (C9481). From Acros Organics: Ethylenediaminetetraacetic acid, disodium salt dihydrate (EDTA) (147850010).

### Eutectic characterization

The melting points of eutectic systems can be characterized visually, but differential scanning calorimetry (DSC) offers a much more accurate determination of melting point, and can be used to determine more complex phase diagrams. A eutectic system will have a single eutectic point - a specific ratio of the two components that will form a uniform melting mixture - which appears as a sharp melt peak on DSC (FIG. 1(c)) similar to a pure component peak (FIGs. 1(a) & 1(e)). If the ratio of components differs from the exact eutectic ratio, the eutectic peak will still appear (and melt at the same temperature), but will be followed by a second, asymmetrical peak as the component that is not in the eutectic gradually starts to melt. The farther the ratio of components is away from the eutectic ratio, the higher the melting temperature of this second "liquidus" peak will be (FIG. 1(b)). This system also exhibits an incongruently melting solid phase, visible before the eutectic in some mixtures (FIG. 1(d)) Graphing these points on a Txy chart will give the phase diagram of the two component system. For pure component and eutectics, Tₘ is the onset of melting (Tₒₙₛₑₜ), while for the liquidus, it is the temperature at the tip (Tₚₑₐₖ).

Referring now to FIG. 1, the figure illustrates DSC thermograms for determination of Lidocaine:Benzocaine eutectic system: a) pure lidocaine with a single melt peak; b) 80% lidocaine, 20% benzocaine, the left peak is the melting of the eutectic, the right is the liquidus; c) lidocaine:benzocaine eutectic (65% lidocaine) showing only one peak; d) equal mass mixture of lidocaine and benzocaine, showing an incongruent melting solid, followed by the eutectic and (small) liquidus melting peaks; and e) pure benzocaine melt peak. The Y-axis was scaled for each run to ensure visibility of all peaks, so peak sizes are not comparable between runs.

Since the specific crystalline or glassy state of a solid can affect its melting temperature, best practices in DSC involve repeated melt/thaw cycles with a single sample, where presumably the freezing and melting peaks will be at the same temperature and of equivalent size (enthalpy of fusion is the same whether melting or freezing, just with opposite sign). However, in the absence of a nucleation point, some of the eutectic melts studied here can form supercooled liquids. For example, lidocaine:benzocaine combinations near the eutectic point (65:35, Tₘ~22°C) may remain liquid for weeks at 4°C or days at -20°C.

To overcome the stability of the supercooled liquid, components were mixed in various ratios, heated to above the melting point of the higher-melting ingredient until a clear liquid formed, then flash frozen at -80°C overnight so that crystallization would begin, before moving to -20°C for a week to allow for complete freezing. The resulting solids were crushed into fine powders in a chilled mortar and pestle and stored at -20°C until ~10mg were weighed into chilled Tzero hermetic sample pans (TA). Pans were quickly transferred to the DSC, and rapidly cooled (20°C/min) to below 0°C, held there for 2 minutes, then ramped at 10°C/min to above the highest melting point. A sealed empty pan was used as reference. This method was based on one used to characterize the lidocaine:menthol system. Note that this pre-melting and fast freezing method may, due to kinetic limitations, determine only a "metastable" phase diagram, rather than true stable crystalline states. Reassuringly, for the lidocaine:menthol system, both the metastable and "true" stable phase methods identified the eutectic point, with most disagreements limited to the phase diagram far away from this ratio.

### Viscosity measurements

Viscosity of individual components and full formulations was measured on a Brookfield RS-Plus rheometer (AMETEK Brookfield, Middleboro, MA) with RC3-25-1 cone. Approximately 300 uL of sample was placed on the stage before lowering the cone, and excess sample was scraped away from the sides of the cone. Measurements were taken over a range of temperatures (5 to 37°C) to detect temperature-dependent Pluronic gelation. Due to the shear-thinning properties of polymers, viscosity was measured at multiple shear rates (5, 50, 500, and 2000 s⁻¹) when samples contained CMC or Pluronic. Samples with viscosities too low for measurement are reported as the lower limit of the RC3-25-1 measuring system (0.06 Pa-s). Where appropriate, more concentrated stocks were substituted to bring viscosity within a measurable range, such as the use of 5% CMC in place of 2% in the shear-thinning analysis.

### Eutectic mixtures

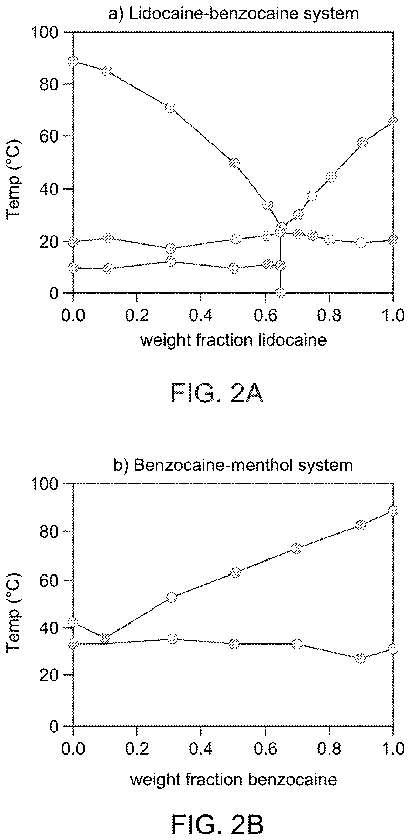

The lidocaine:benzocaine eutectic point occurs at the ratio 65:35, and melts at 22°C (FIG. 2a). In contrast to the simplest eutectic systems, where only the eutectic and liquidus peaks are present, a third peak appears in this system when lidocaine is less than 65%, indicating the presence of an incongruently melting solid phase.

Referring now to FIG. 2, the figure illustrates phase diagrams of a) Lidocaine:benzocaine and b) benzocaine:menthol eutectic systems. Dots represent peaks on DSC thermograms (data), lines represent approximate phase boundaries (interpolation).

The melted eutectic is a clear Newtonian liquid, immiscible with and slightly denser than water, and several hundred times more viscous (180 mPa-s at 25°C). If the eutectic and water are mixed in equal volumes and allowed to separate, the drug-saturated aqueous phase becomes alkaline, and contains approximately twice as much lidocaine as benzocaine (due to its higher solubility). The water-saturated eutectic exhibits reduced viscosity (80 mPa-s at 25°C) and a lower melting temperature (14°C) than the dry eutectic. Hydrophobic small molecules preferentially partition into the oily eutectic, to the point where 0.5 g/L bupivacaine-HCl, 5 g/L chlorobutanol, and 25 g/L benzyl alcohol were all undetectable in the aqueous phase after 10 minutes of mixing with the eutectic. The liquid eutectic appears more stable than aqueous solutions of lidocaine or benzocaine, although a yellow-brown color may start to appear if exposed to bright light for long periods of time.

The eutectic point of benzocaine:menthol occurs at 10:90 and 34°C (FIG. 2b). Therefore, this eutectic is mostly menthol, and melts only slightly below pure menthol (42°C), so treating this as a solvent-solute system where menthol melts and then benzocaine dissolves in is intuitive.

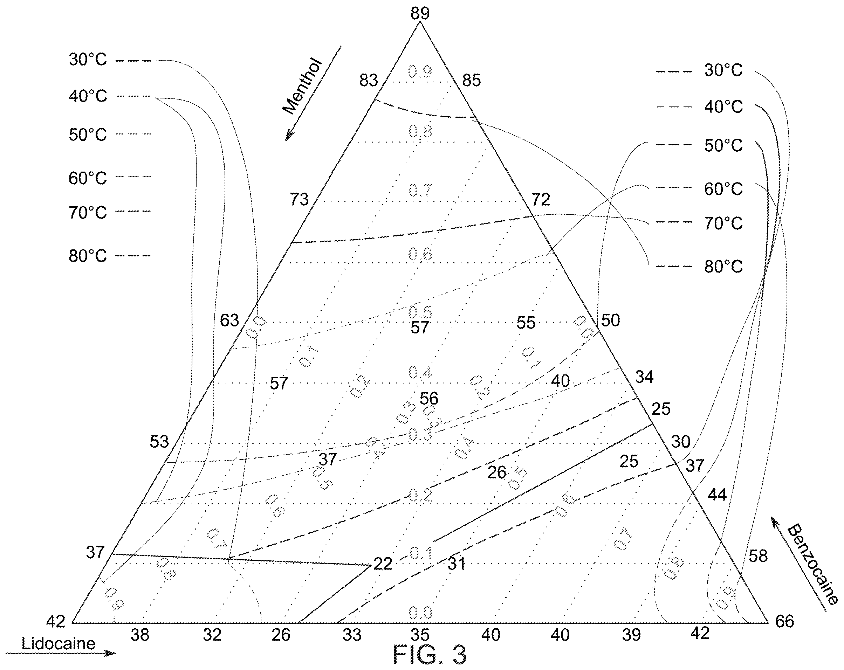

Since lidocaine and benzocaine form a eutectic with each other, and both also form eutectics with menthol (the lidocaine:menthol eutectic point is 30:70, Tₘ=26°C), a three component eutectic point between the three also exists. DSC runs of the three component mixtures contain additional peaks which are not always easy to classify, and graphing the multiple lines of eutectic, liquidus, and various incongruent melting components is difficult in three dimensions. Typically, a single phase transition, such as the final liquidus line indicating a fully melted homogeneous liquid, can be shown as lines connecting points that melt at the same temperature. These isotherms (dotted lines in FIG. 3) each denote a slice through the three dimensional system at a single temperature.

As expected, the ternary phase diagram indicates low melting temperatures along the "troughs" between the individual two-component eutectic points (black lines in FIG. 3), with a large region of mixtures that melt below 30°C running between the lidocaine:benzocaine and lidocaine:menthol binary eutectic points. While the three component system does not contain any points melting below the lidocaine:benzocaine eutectic point of 22°C, the inclusion of menthol in the system expands the range of anesthetic ratios where room temperature melting is possible. Small amounts of menthol appear to inhibit crystal formation with 67.5:27.5:5% LiBeMe mixtures remaining liquid indefinitely at 4°C (this is the ratio used in the LBB-111 formulation). Water saturated LiBeMe exhibits the same reduced viscosity and lower melting point as the two-component lidocaine:benzocaine system.

Referring now to FIG. 3, FIG. 3 illustrates a phase diagram of the lidocaine:benzocaine:menthol ternary system. Black numbers within the triangle (and along edges) indicate liquidus points of the given mixture, in °C (real data). Colored dashes are estimated isotherm lines based on the measured melting points. Solid black lines approximate the low melting "troughs" between the two-component eutectic values along the axes.

### Formulation viscosity

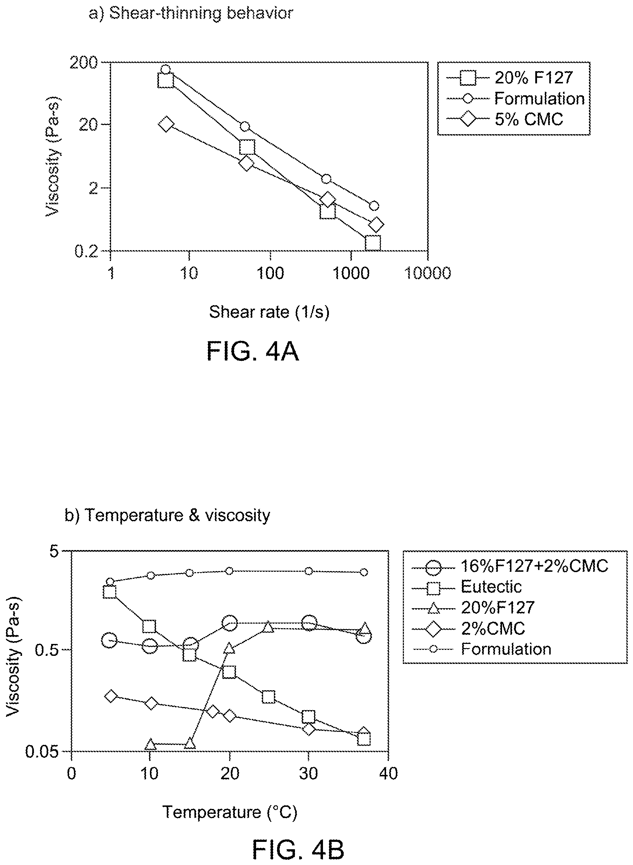

Aqueous solutions of Pluronic F127 behave as Newtonian liquids at low temperatures, but heating above the solution's gel temperature (T_{g}, varies with concentration) causes micelles to form, creating a viscous shear-thinning gel. Carboxymethylcellulose solutions also exhibit shear-thinning behavior, but reduced viscosity with increasing temperature. Viscosity of the pure eutectic melt is Newtonian and declines with increased temperature. The final anesthetic formulation containing eutectic emulsified in Pluronic F127 and CMC has a viscosity that remains relatively constant over a wide range of possible storage temperatures (5 to 37°C), but varies significantly with shear rate (FIG. 4A).

Referring now to FIG. 4, FIG. 4 illustrates graphs depicting the viscosity of components and final anesthetic formulation. a) The effect of different shear rates on viscosity of the polymer additives and LBB-111 formulation, all at 37°C. b) The effect of temperature on viscosity of the various components as well as the LBB-111 formulation, all measured at a shear rate of 500/s. The LBB-111 formulation contains by weight 16% F127, 2% CMC, 20% Eutectic (67.5% lidocaine, 27.5% benzocaine, 5% menthol), as well as 0.5% Tween 80 and 0.05% EDTA.

### Stability of drugs in solution and in the Anesthetic formulation

To test the stabilities of the three drugs in solution, all three were dissolved together in 0.5x saliva analogue buffer to final concentrations equivalent to complete dissolution of 1g of the formulation in one liter of buffer (0.124 g/L lidocaine, 0.053 g/L benzocaine, and 0.009 g/L bupivacaine HCl), then aliquots were stored in a 4°C refrigerator (Fridge), 40°C incubator (Heat), or on the benchtop at room temperature exposed to light (Light). Identical samples were prepared with 0.5g/L EDTA added as preservative). These aliquots were quantified by HPLC, alongside aliquots of the formulation (both with and without EDTA) treated in the same manner, which were dissolved in ethanol immediately before quantification.

Lidocaine, benzocaine and bupivacaine were detected on a Shimadzu HPLC system consisting of LC-20AT solvent delivery unit with DGU-20A in-line degasser, SIL-20A HT autosampler, CTO-20AC column oven, SPD-M20A PDA detector, and CBM-20A system controller. The column was a YMC-Pack Pro C18 column (3 µm particle size, 120 Å pore size, 4.6 x 150 mm) and mobile phase was 30 mM potassium phosphate, 0.16% trimethylamine, adjusted to pH 4.9 with phosphoric acid, 30% Acetonitrile.

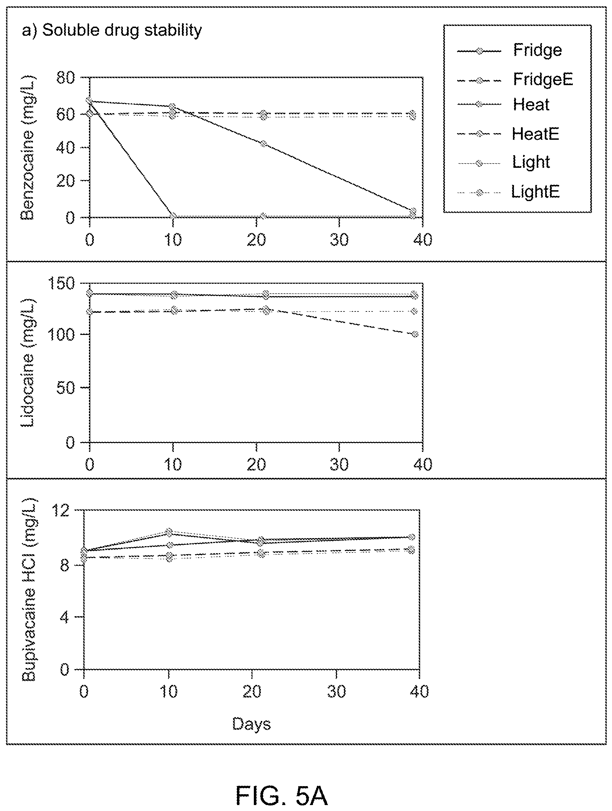

The results for drugs in solution (FIG. 5a) indicate that benzocaine is substantially less stable than the other two drugs, becoming undetectable within 10 days under light or heat conditions, but that EDTA could completely eliminate this degradation (the slight dilution caused by initial addition of the EDTA solution is also visible). Degradation was almost undetectable for both lidocaine and benzocaine, although the Heat sample with EDTA did show some lidocaine degradation after over one month. To confirm that the HPLC method could detect degradants of lidocaine and benzocaine, a forced degradation study was carried out. It confirmed that lidocaine and benzocaine are quite stable in the presence of light, heat, and acid, but did degrade under oxidizing conditions (3% hydrogen peroxide).

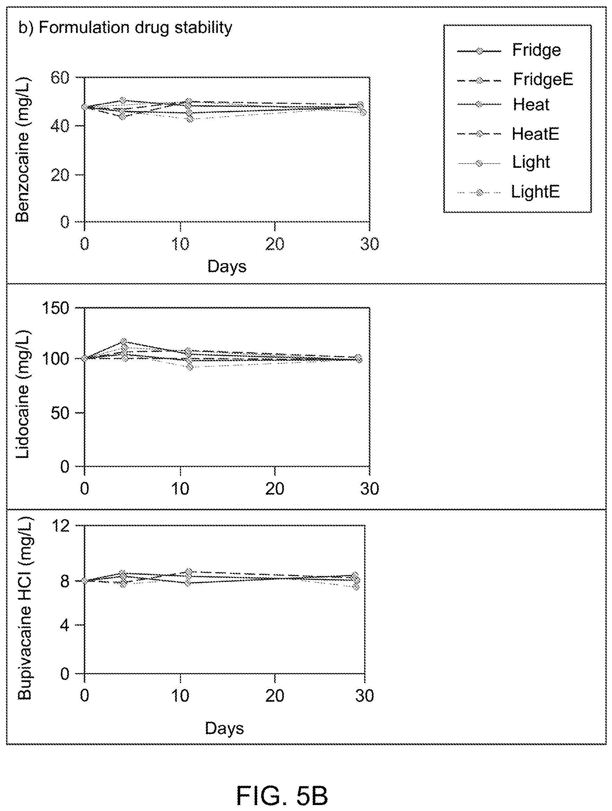

The formulation data is noisier (probably reflecting variability in the ethanol solubilization step), but indicates that all three drugs are stable in the formulation, regardless of the addition of EDTA (FIG. 5b). The lack of benzocaine degradation in the formulation even without EDTA likely results from improved stability when sequestered in the oily (eutectic) phase of the emulsion. The fact that the emulsion is opaque may also protect against photodegradation.

Referring now to FIG. 5, FIG. 5 illustrates a series of graphs depicting the stability of example drugs (e.g., benzocaine, lidocaine, and Bupivacaine HCl) a) dissolved in saliva analogue buffer, b) in the eutectic emulsion formulation. "Fridge" samples stored at 4°C, "Heat" at 40°C, "Light" on bench in clear vials. An "E" after the storage condition indicates samples with 5g/L EDTA added as preservative.

Eutectic melts of local anesthetics may demonstrate various advantages from a formulation standpoint, but may be limited by the inherent eutectic point of the chemicals in question. For example, the lidocaine:menthol eutectic is of reduced utility because the 80% menthol content provides minimal anesthetic effect, while contributing a potentially overwhelming taste and smell. Therefore, the natural eutectic point of the lidocaine:benzocaine system, consisting of 65% lidocaine, is advantageous because it allows for more potent formulations.

Carboxymethylcellulose is an effective hemostatic agent, and while much of this can be attributed to physical effects (absorption of water, gel viscosity), it may also play a role in accelerating clotting. Given the importance of maintaining a stable clot for averting dry socket, the inclusion of a clotting aid in the formulation is important.

One challenge for the study of the efficacies of these topical anesthetic eutectic systems in animals and humans has been a shortage of pharmaceutical grade reagents. Many of the drugs in this class exist as both free-base and ionic salts, but only the pure base can form a eutectic. Lidocaine-tetracaine eutectic formulations were developed as an in-house alternative to EMLA because pharmaceutical grade free-prilocaine was commercially unavailable. Likewise, pharmaceutical grade bupivacaine can only be purchased as the HCl salt, so in cases where the free base is needed, it must be purified from the salt by alkaline extraction.

Various embodiments of the disclosure have been described in fulfillment of the various objects of the disclosure. It should be recognized that these embodiments are merely illustrative of the principles of the present disclosure. Numerous modifications and adaptations thereof will be readily apparent to those skilled in the art without departing from the spirit and scope of the disclosure.

### EXAMPLE 2 - Release Properties

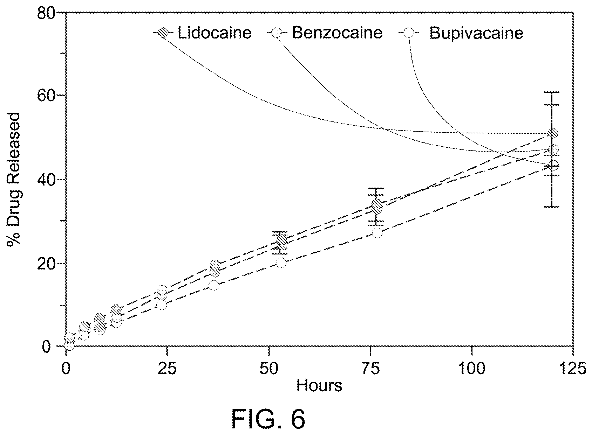

As a first step to approximate release properties of LBB-111, we performed *in vitro* dissolution testing under various conditions. Dissolution testing was carried out in a Vision Elite 8 dissolution apparatus with 1L round bottomed covered vials. Mixing was accomplished using the smaller "mini" spin paddles at 59 rpm. Dissolution media [900 mL Phosphate buffered saline (PBS), pH 7.4] was equilibrated at 37°C overnight before the start of a run. To mimic a human molar socket, cylindrical chambers with internal dimensions approximately 1 cm tall and 1 cm in diameter, with a high infill base to give the socket weight and stability were 3D printed at the UNC BeAM Makerspace using an Ultimaker 3. Sockets were filled to the rim with formulation using an 18 g needle, and the formulation weight (around 0.8 g for these sockets) was recorded. The flat base of the socket was placed on the bottom of each dissolution chamber and mixing initiated. Two milliliter samples were taken at regular intervals up to 120 hours and concentrations of lidocaine, benzocaine, and bupivacaine determined by HPLC. These studies demonstrated that (i) all three anesthetic agents are released into the dissolution media over a period of up to 5 days (120 hours), (ii) individual drugs are released at subtly different rates, with benzocaine the fastest, closely followed by lidocaine, and bupivacaine the slowest **(****Fig 6****).** This order roughly matches the expected onset and relative duration of each drug, (iii) dose dumping was not observed, and (iv) by day five, less than 60% of each drug was released to the media, suggesting that drug release from LBB-111 may extend and additional 2-3 days at these experimental conditions.

Referring now to FIG. 6, the graph illustrates drug released into PBS medium plotted as the fraction of the total that was present in the formulation at the start (% released). Each formulation contained three drugs: Lidocaine, Benzocaine and Bupivacaine. Formulation samples were dissolved in 19-times their weight (final conc. 5% wt) 200-proof ethanol, the ethanol dissolved samples were then diluted 5-fold into distilled water, and 10 ilL of this dilution was injected.

The following disclosures were disclosed in the parent application and repeated here to provide basis for amendment or further divisional application as required.

### DISCLOSURES

1. An anesthetic emulsion comprising:
   a dispersed phase comprising a eutectic mixture of lidocaine and benzocaine; and
   an aqueous or aqueous-based continuous phase comprising bupivacaine.
2. The anesthetic emulsion of disclosure 1, wherein a ratio of lidocaine to benzocaine ranges from 1.5 to 3.
3. The anesthetic emulsion of disclosure 1, wherein the aqueous phase further comprises one or more gelling agents.
4. The anesthetic emulsion of disclosure 1, wherein the eutectic mixture further comprises menthol.
5. The anesthetic emulsion of disclosure 1, wherein the lidocaine is present in an amount of 10-20 weight percent of the anesthetic emulsion.
6. The anesthetic emulsion of disclosure 1, wherein the benzocaine is present in an amount of 3-8 weight percent of the anesthetic emulsion.
7. The anesthetic emulsion of disclosure 4, wherein the menthol is present in an amount of 0.5-2 weight percent of the anesthetic emulsion.
8. The anesthetic emulsion of disclosure 3, wherein the one or more gelling agents are present in a total amount of 15-25 weight percent of the anesthetic emulsion.
9. The anesthetic emulsion of disclosure 1 having a viscosity of 3-5 Pa-s at 30°C and a shear rate of 500/s.
10. The anesthetic emulsion of disclosure 3, wherein the one or more gelling agents comprises a block copolymer comprising hydrophobic and hydrophilic blocks.
11. The anesthetic emulsion of disclosure 1, wherein the bupivacaine is present in an amount up to 5 weight percent of the anesthetic emulsion.
12. A method of treating a site of damaged tissue comprising:
   applying an anesthetic emulsion to the site of damaged tissue, the anesthetic emulsion comprising a dispersed phase comprising a eutectic mixture of lidocaine and benzocaine, and an aqueous or aqueous-based continuous phase comprising bupivacaine.
13. The method of disclosure 12 further comprising releasing at least one of the lidocaine, benzocaine, and bupivacaine to the site of damaged tissue over a period of at least three days.
14. The method of disclosure 12, wherein the site of damaged tissue is a surgical site.
15. The method of disclosure 14, wherein the surgical site resides in a patient's mouth.
16. The method of disclosure 15, wherein the surgical site is a tooth socket.
17. The method of disclosure 12, wherein the site of damaged tissue is a site of injury.
18. The method of disclosure 17, wherein the site of injury comprises one or more lacerations.
19. The method of disclosure 12, wherein a ratio of lidocaine to benzocaine ranges from 1.5 to 3.
20. The method of disclosure 12, wherein the eutectic mixture further comprises menthol.
21. The method of disclosure 12, wherein the aqueous phase further comprises one or more gelling agents.
22. The method of disclosure 12, wherein the anesthetic emulsion has a viscosity of 3-5 Pa-s at 30°C and a shear rate of 500/s.

## Claims

1. An anesthetic emulsion comprising:
a dispersed phase comprising a eutectic mixture of menthol, lidocaine, and benzocaine; and
an aqueous or aqueous-based continuous phase comprising bupivacaine.

2. The anesthetic emulsion of claim 1, wherein a weight ratio of lidocaine to benzocaine ranges from 1.5 to 3.

3. The anesthetic emulsion of claim 1, wherein the aqueous phase further comprises one or more gelling agents.

4. The anesthetic emulsion of claim 1, wherein the lidocaine is present in an amount of 10-20 weight percent of the anesthetic emulsion.

5. The anesthetic emulsion of claim 1, wherein the benzocaine is present in an amount of 3-8 weight percent of the anesthetic emulsion.

6. The anesthetic emulsion of claim 1, wherein the menthol is present in an amount of 0.5-2 weight percent of the anesthetic emulsion.

7. The anesthetic emulsion of claim 3, wherein the one or more gelling agents are present in a total amount of 15-25 weight percent of the anesthetic emulsion.

8. The anesthetic emulsion of claim 1 having a viscosity of 3-5 Pa-s at 30°C and a shear rate of 500/s.

9. The anesthetic emulsion of claim 3, wherein the one or more gelling agents comprises a block copolymer comprising hydrophobic and hydrophilic blocks.

10. The anesthetic emulsion of claim 1, wherein the bupivacaine is present in an amount up to 5 weight percent of the anesthetic emulsion.

11. An anesthetic emulsion as claimed in any of claims 1 to 10 for use in treating a site of damaged tissue, the use comprising:
applying said anesthetic emulsion to the site of damaged tissue

12. The anesthetic emulsion for use according to claim 11, the use further comprising releasing at least one of the lidocaine, benzocaine, and bupivacaine to the site of damaged tissue over a period of at least three days.

13. The anesthetic emulsion for use according to claim 11, wherein the site of damaged tissue is a surgical site.

14. The anesthetic emulsion for use according to claim 13, wherein the surgical site resides in a patient's mouth, optionally wherein the surgical site is a tooth socket.

15. The anesthetic emulsion for use according to claim 11, wherein the site of damaged tissue is a site of injury, optionally wherein the site of injury comprises one or more lacerations.
